# EUROPEAN PATENT APPLICATION

(11) **EP 0 958 798 A1**
(43) Date of publication of application: **24.11.1999**
(21) Application number: 99500077.5
(22) Date of filing: 12.05.1999
(51) Int. Cl.: A61F 5/08

(54) **Nasal anatomical device to increase the respiratory capacity.**

(30) Priority: 22.05.1998 ES 9801330 U
(71) Applicant: Guerra Navas, Antonio, E-01007 Vitoria (Alava) (ES)
(72) Inventor: Guerra Navas, Antonio, E-01007 Vitoria (Alava) (ES)
(74) Representative: Davila Baz, Angel

(57) **Abstract**

Nasal anatomical device to increase the respiratory capacity, which is constituted by two identical open loops, of annular shape and constructed of an elastically deformable material, which is aseptic and antiallergenic, and linked by a U-shaped bridge-piece, which is also elastically deformable.

## Description

The present invention refers to a nasal anatomical device to increase the respiratory capacity, conceived to be introduced in the nostrils so as to induce an opening of the same in relation to their usual position, thus providing an increase in the respiratory capacity.

The possibility of increasing the respiratory capacity is enormously useful in certain situations, essentially in those in which a considerable physical exercise is performed and which require an increase in the volume of air which reaches the lungs as it happens when practising any sporting activity.

The object of the present invention is that of proposing a device by means of which it may be possible to achieve an opening of the nostrils, in relation to their usual position, with the aim of allowing a greater flow of air into the lungs, improving in this manner the respiratory capacity.

According to the present invention, the device is constituted by two identical open loops and a linking bridge-piece between them.

The open loops have an annular configuration and are constructed in an elastically deformable material which is also aseptic and antiallergenic, being said loops so shaped as to be tightly introduced into the nostrils by partially closing them, by elastic deformation, exerting a gentle radial pressure along the periphery of the loops.

The linking bridge-piece which relates and binds the two open loops is constituted by a U-shaped clamp, which is also capable of being elastically deformed. Each one of the open loops is attached to the end of one of the arms of the bridge-piece, in a considerably perpendicular position to said arms, so that the loops are situated in a symmetrical position, according to planes which form an obtuse angle, being the opening of both loops preferably oriented in the same direction.

The annular loops and the bridge-piece can both be of different sizes, so that the device may adjust, as far as possible, to the anatomical characteristics of the user.

Each one of the annular loops is introduced into one of the nostrils, prior partially closing the loops, causing an opening of the same which is greater than the usual one, by virtue of its elastic properties.

The flexibility and elasticity of the materials will be such as to guarantee the opening of the nostrils, but which will give up and deform in the event of impact upon the nose, so that no harm will be caused to the user as a consequence of its use.

The material employed for the manufacture of the device will be totally aseptic and antiallergenic, of similar features to that used in prosthetics and surgical devices.

The device shall have a rounded, anatomically designed shape, absolutely lacking any edges which may produce some kind of harm to the inside of the nostrils. For the same reason, the surface finishing of the material will be of such nature as to ensure the absence of edges or overflush.

Optionally, the device may be accompanied by a small plastic box, to protect it from the dirt and keep it isolated while it is not being used.

The characteristics and advantages of the object of the invention will be better understood with the aid of the following description, made by referring to the enclosed figures in which a non-limitating example of an embodiment of the invention is shown.

In the figures :

Figure 1 is a perspective of a nasal anatomical device, constituted according to the invention.

Figure 2 is a front view of the device in figure 1.

Figure 3 is a plan view of the same device.

Figure 4 is a front view of the device placed in the nostrils of a user.

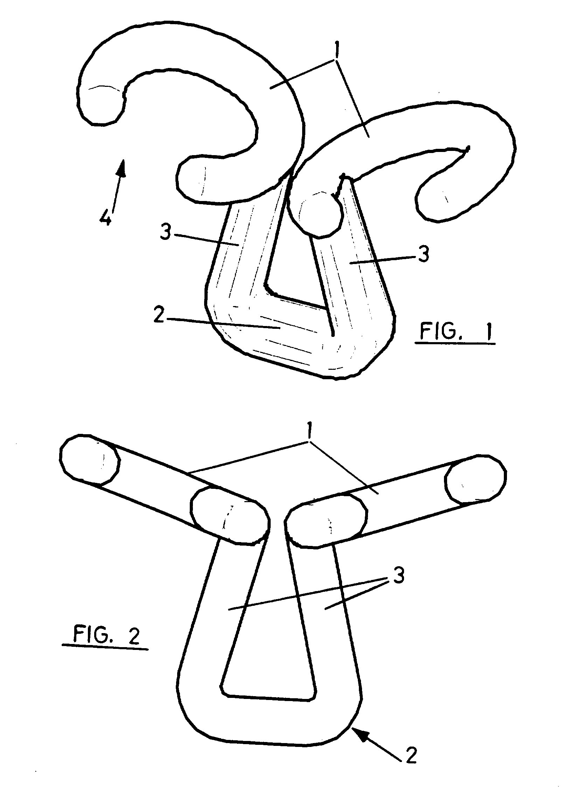

As can be appreciated in figures 1 to 3, the device of the invention is constituted by two open annular loops, labelled as 1, which are related to each other by means of a bridge-piece 2.

Loops 1 are constituted by an elastically deformable material, aseptic and antiallergenic. The loops are sized in such as manner that, when they are partially closed, by means of a gentle radial compression, they can be introduced inside the nostrils.

The bridge-piece 2 is shaped as a U-shaped clamp, having convergent side arms 3, being each one of the loops 1, attached in a position which is perpendicular to the end of one of arms 3. The loops 1 are thus placed in a position symmetrical with each other, forming an obtuse angle, as can be seen in figure 2.

The loops 1 are open in a portion or section 4, which is of a sufficient width so as to allow a sufficient reduction in the contour of said loops in order to facilitate their introduction into the nostrils.

The bridge-piece 2 shall be elastically deformable so that the loops 1 may be easily approximated or separated.

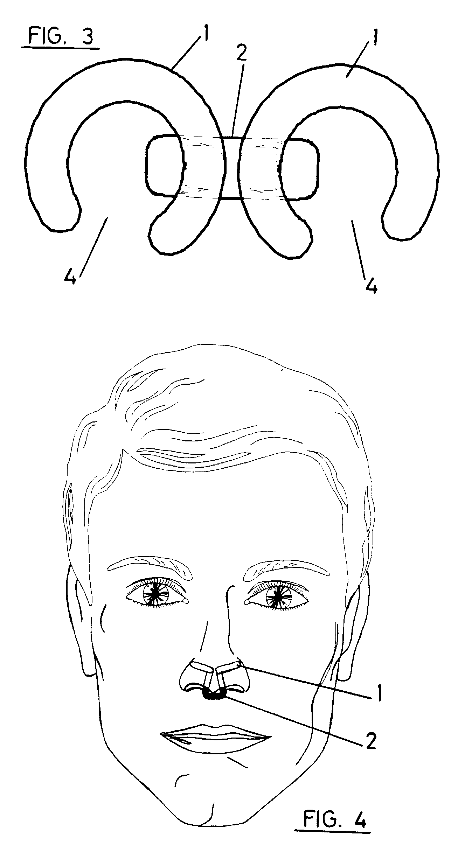

With the described design, the proper placing of the device of the invention is achieved by gently compressing the loops 1 so that they become partially closed, and thus, their contour is reduced. In this position they are introduced into the nostrils, as shown in figure 4, with the nasal septum remaining between the arms 3 of the bridge-piece 2. When the loops 1 are released they recover their initial diameter, bringing about the dilation or widening of the nostrils, and with it, a greater capacity for the penetration of air.

## Claims

1. Nasal anatomical device to increase the respiratory capacity, characterised in that it is constituted by two identical open loops (1) and by an intermediate linking bridge-piece (2); being said loops (1) of an open annular shape, constructed of an elastically deformable material which is aseptic and antiallergenic, and which are sized for their tight introduction into the nostrils by means of their partial closure, by radial elastic deformation; and the bridge-piece (2) which is constituted by an elastically deformable U-shaped clamp, being one of the loops attached to the end of each of its arms (3), remaining the loops in a symmetrical position.

2. A device according to claim 1, characterised in that the loops (1) are attached to the ends of the arms (3) of the bridge-piece (2) in positions which are considerably perpendicular to said arms (3), both loops (1) remaining in planes which are at an obtuse angle with each other and with the opening directed in the same sense.
